Europäisches Patentamt

European Patent Office

Office européen des brevets

(11) Publication number : **0 537 105 A1**

# (12) EUROPEAN PATENT APPLICATION

(21) Application number : **92810549.3**

(22) Date of filing : **17.07.92**

(51) Int. Cl.⁵ : **C12N 15/75,** C12P 21/00,
C12N 15/32, A01N 63/00,
// (C12P21/00, C12R1:07)

(30) Priority : **26.07.91 US 736668**

(43) Date of publication of application :
**14.04.93 Bulletin 93/15**

(84) Designated Contracting States :
**AT BE CH DE DK ES FR GB GR IT LI LU NL PT
SE**

(71) Applicant : **SANDOZ LTD.**
**Lichtstrasse 35**
**CH-4002 Basel (CH)**
(84) **BE CH DK ES FR GB GR IT LI LU NL PT SE**

(71) Applicant : **SANDOZ-PATENT-GMBH**
**Humboldtstrasse 3**
**W-7850 Lörrach (DE)**
(84) **DE**

(71) Applicant : **SANDOZ ERFINDUNGEN**
**VERWALTUNGSGESELLSCHAFT M.B.H.**
**Brunner Strasse 59**
**A-1235 Vienna (AT)**
(84) **AT**

(72) Inventor : **Gamel, Pamela Hope**
**13055 Dahlia Circle 114**
**Eden Prairie, MN 55344 (US)**
Inventor : **Piot, Jean-Christophe**
**1328 Orange Avenue**
**Menlo Park, CA 94025 (US)**

(54) **Vectors for bacillus thuringiensis.**

(57) Origins of replication from Bacillus thuringiensis kurstaki HD73 which are useful in preparing vectors for use in stably transforming Bacillus thuringiensis strains without displacement of their native plasmids.

EP 0 537 105 A1

# EP 0 537 105 A1

Field of the Invention

This invention relates to origins of replication isolated from <u>Bacillus thuringiensis</u> plasmids, plasmid vectors containing these origins of replication, their use in cloning and the transformed strains obtained thereby.

Background to the Invention

Biological insecticides in general and <u>Bacillus thuringiensis</u> (B.t.) based products in particular have in recent years found increasing use as specialised alternatives to broad spectrum chemical pesticides due to their occurrence in nature and their high host specificity.

The increased acceptance of such products has at the same time led to more sophisticated demands with regard to spectrum of activity against target pests and ability to spare non-target insects.

Various methods of achieving this result are known such as isolation of strains containing mutated toxin-encoding genes where the mutation may be spontaneous or induced (cf GB Patent Appln. 2216127A) or conjugation of naturally occurring strains or mutated strains to produce hybrid strains containing plasmids from both parental strains having differing or complementary host specificities (cf USP 4,797,279). Each of these methods suffers from certain drawbacks and limitations such as the unpredictability of mutation and inability of all strains to act as donors and of all plasmids to successfully transfer from one strain to another.

This has led to increased use of recombinant DNA techniques to introduce genes expressing crystal protein toxins or other desired proteins into host cells in a flexible and controlled fashion. For example transformation of <u>B.t.</u> strains using techniques such as electroporation or electrotransformation is finding increasing use (cf Bore & Ellan, FEMS, Microbiology Letters 58 (1989) 171-178; GB Patent Appln. 2219806A and EP Patent Appln. 433945A; Baum et al. Appl. Environ. Microbiol. v 56 (11) 3420-28, Nov 1990).

As discussed in detail in the above references an essential element in successful manipulation of <u>B.t.</u> strains to beneficially affect the production, regulation, activity, (degree/spectrum) and the like of the crystal protein toxins or to introduce other desirable protein genes is the availability of suitable cloning vectors for transfer of the desired genetic material into the host strain.

A general discussion of the nature and purpose of vectors used to date in the transformation of <u>B.t.</u> is contained in the above citations and in Miteva et.al. (1988 Arch. Microbiol. <u>150</u>:496-498).

Until now, however, successful transformation of <u>B.t.</u> strains to introduce heterologous genetic material had employed plasmid vectors containing in addition to DNA from <u>B.t.</u> significant amounts of DNA from heterologous organisms such as <u>E. coli</u> (cf EP 433945A), <u>Bacillus cereus</u> (cf GB 2219806A) and the like. The presence of DNA from heterologous organisms can lead to stability problems within the host strain and also it would clearly be more desirable to have transformed organisms containing predominantly or solely DNA native to their species.

Baum <u>et al</u>. (Appl. Environ. Microbiol. v 56 (II) 3420-28, Nov 1980) have described the cloning in <u>E. coli</u> of seven replication origins from resident plasmids of <u>Bacillus thuringiensis kurstaki</u> (B.t.k.) HD 263 and HD 73. Three of these origins, originating from the 43, 44 and 60 MDa plasmids of <u>B.t.k.</u> HD 263, were used to construct a set of shuttle vectors. However, each of these cloned replication origins exhibits incompatibility with the resident <u>B.t.</u> plasmid from which it was derived (Baum & Gilbert (1991),J. Bacteriol. <u>173</u> (17) 5280-5289).

Baum <u>et al</u>. (ibid) also describe the cloning of plasmid DNA fragments containing origins of replication from <u>B.t.k.</u> HD73-26-10 a transconjugant strain which harbours the 44 MDa resident plasmid of HD263 and a 4.9 MDa plasmid of unspecified source. Novel plasmids pEG588-2, pEG588-18 and pEG588-21 were obtained which hybridised strongly on Southern blots to a hybridisation probe consisting of the 4.9 MDa plasmid of strain HD73-26. However, HD73-26 transformants of these plasmids showed a reduction in, or absence of, the resident 4.9 MDa plasmid of HD73-26, suggesting that these novel plasmids exhibit some degree of incompatibility with the 4.9 MDa plasmid.

We have now found that it is possible to construct DNA vectors which are stable in <u>B.t.</u> host cells and contain predominantly <u>B.t.</u> DNA. Further, by appropriate choice of source plasmid for the origin of replication and manipulation of the DNA thereof, we have found that it is possible to provide plasmids which are compatible with the natural resident plasmids from which the origin of replication is derived.

Description of the Invention

Accordingly, in a first aspect, the present invention provides a DNA vector for use in the transformation of <u>Bacillus thuringiensis</u> cells, comprising an origin of replication from <u>Bacillus thuringiensis kurstaki</u> HD73 substantially free of DNA with which it is normally associated and which is not necessary for replication.

The origin of replication may be from any <u>B.t.k.</u> HD73 plasmid. Preferably the origin of replication is from the 5.2 MDa, 5.6 MDa or most preferably from the 50 MDa plasmid of HD73. The DNA sequence of the minimal origin of replication of the 50 MDa plasmid is given hereinafter as SEQ ID NO:6 in the SEQUENCE LISTING.

In addition to the natural HD73 plasmid origins of replication, e.g. the origin of the 50 MDa plasmid, homologue DNA sequences may be used which have deletions, substitutions or minor additions, e.g. insertions, with respect to the natural origin DNA sequence which do not affect the ability of the sequence to function as an origin of replication.

Preferably also the DNA vector of the invention contains predominantly B.t. DNA.

The term predominantly B.t. DNA refers to vectors which contain little or no DNA from other cells with the exception of multiple cloning sites, genes required as markers or coding for proteins desired for incorporation into host cells.

A particular advantage of employing an origin of replication from one of the HD73 plasmids is that removal of DNA responsible for functions other than replication enables plasmid vectors to be constructed which may be stably introduced into strains containing resident plasmids with similar origins of replication without displacing these resident plasmids from the transformed strain. In this way it is possible, for example, to introduce genes encoding heterologous crystal protein toxins or other desired proteins into HD73 using a vector such as described hereinafter comprising the origin of replication derived from the 50 MDa plasmid of HD73 without displacing the full 50 MDa plasmid contained therein naturally which carries the important cry IA(c) crystal protein toxin gene. The 50 MDa plasmid from HD73 is known, e.g. from Kronstad and Whiteley, (J. Bacteriol. 160:95-102). However the origin of replication of this plasmid has not been the subject of previous detailed investigation or discussion.

Vector construction and/or transformation of B.t. strains may be carried out in a conventional manner, for example as described in the references cited above or in Lereclus et.al. (FEMS Microbiology Letters 60 (1989) 211-218); McDowell et.al. (Plasmid 25:113-120, 1991) or Hardy (Plasmids: A Practical Approach Washington, D.C. IRL Press, 1987).

Suitable vectors for use in the present invention may comprise in addition to an origin of replication derived from an HD73 plasmid, one or more of: a means for selecting hosts transformed by the plasmid, e.g. a gene encoding a selectable marker such as a gene conferring antibiotic resistance; a cloning site, preferably a multiple cloning site; a region of DNA which enables gene expression in the chosen host; and one or more DNA sequences which upon expression encode one or more desired B.t. crystal protein toxins or other proteins of interest, the latter two usually being integrated in the cloning site.

Accordingly, in a second aspect, the invention provides a method of producing in a Bacillus thuringiensis strain a desired exogenous protein, which comprises transforming cells of said Bacillus thuringiensis strain with a DNA vector according to the invention which comprises a gene coding for expression of the desired exogenous protein, and propagating said transformed cells in a suitable manner to effect expression of the gene coding for the desired exogenous protein.

[A general discussion of crystal protein toxins and genes encoding them may be found in Hoefte and Whiteley, Microbiol. Reviews, v. 53 n. 2 pp 242-255 (June 1989).]

In further aspects, the invention also includes Bacillus thuringiensis strains transformed with the vectors of the invention, and insecticidal compositions comprising such transformed Bacillus thuringiensis strains, preferably wherein the vector includes a gene coding for a Bacillus thuringiensis crystal protein toxin.

Moreover, the invention includes an origin of replication isolated from Bacillus thuringiensis HD73, especially the origin from the 50 MDa plasmid, the DNA sequence of which is given hereinafter as SEQ ID NO:6 in the SEQUENCE LISTING, or a homologue therof.

The desired origin of replication may be identified, isolated and used to transform B.t. strains as described below in the following general manner.

Plasmid DNA is isolated from a suitable strain of B.t. HD73 and purified. Isopycnic centrifugation and differential centrifugation may be used for isolation of the desired plasmid eg the 50 MDa plasmid. The plasmid may then be digested with appropriate restriction enzymes and the isolated fragments ligated with a suitably digested E. coli plasmid vector and the ligation mixes used to transform an E. coli strain.

Restriction digestion patterns may be used to identify the subclones containing the desired fragments and a suitable marker gene, erythromycin resistance, may be inserted into these plasmids for use in B.t. transformation.

The Invention is further described in the following non limiting Experimental Summary and Examples which refer to the accompanying drawings.

Brief Description of the Drawings

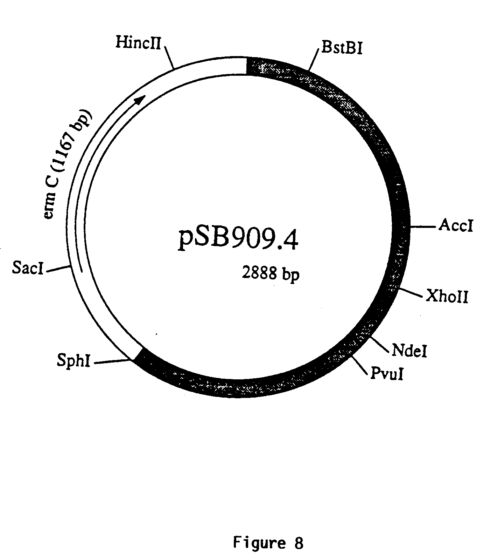

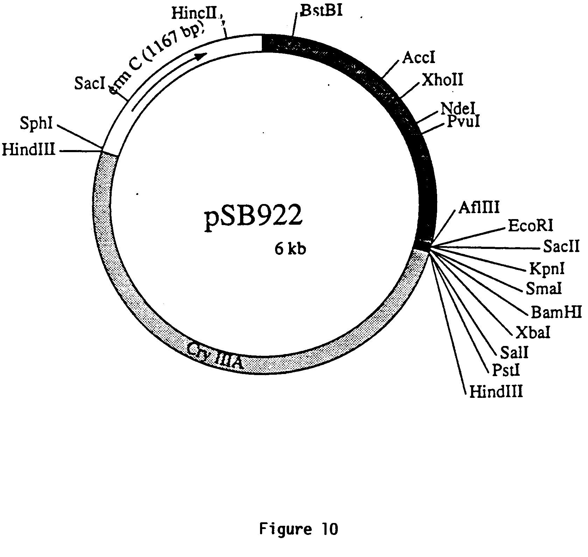

In the accompanying drawings, Figures 1 to 10:

EP 0 537 105 A1

Fig. 1 shows plasmid pSB901 containing an erythromycin resistance gene, ermC;

Fig. 2 shows plasmid pSB904.1 which contains the 9.6 kb Bgl II fragment of the 50MDa plasmid of HD73 and the ermC gene;

Fig. 3 shows plasmid pSB904.2 which is a derivative of pSB904.1 with non-essential portions of the 9.6 kb Bgl II fragment removed;

Fig. 4 shows plsmid pSB904.3 which comprises nucleotides 123-1843 of the 2.4 kb B.t. replicon contained in pSB904.2;

Fig. 5 shows plasmid pSB909 which contains the ermC marker gene and a shortened fragment of the 50MDa plasmid.

Fig. 6 shows plasmid pSB909.3 containing a 2.4 kb portion of the 9.6 kb 50 MDa Bgl II fragment with the ermC gene;

Fig. 7 shows deletion fragments used in determining the minimal replicon from the 50 MDa plasmid of HD73 located on the 2.4 kb portion found on pSB904.1 and pSB909.3;

Fig. 8 shows plasmid pSB909.4 containing the minimal replicon from the 50 MDa plasmid of HD73 together with the ermC gene;

Fig. 9 shows plasmid pSB909.5, comprising this minimal replicon and a multiple cloning site, and

Fig. 10 shows plasmid pSB922 which contains This minimal replicon and the cryIIIA gene from B.t tenebrionis.

## Description of Specific Embodiments of the Invention

### Experimental Summary

Plasmids containing a fragment from the 50MDa plasmid of B.t. kurstaki HD73 together with a selectable marker gene are used to transform a B.t. strain. The transformants showing expression of the marker contain the origin of replication and are selected for further investigation. The selected plasmids are digested with suitable enzymes to remove unwanted DNA sequences and ligated to give a further plasmid containing the desired fragment together with the marker gene which once more may be used to transform B.t. Selective deletion of portions of various plasmids containing the fragment identified as carrying the origin of replication are made using appropriate enzymes and vectors made by ligation of the fragments to cause recircularization. These new constructs are used to again transform B.t. whereby only the construct containing the desired origin of replication will produce identifiable transformants. The desired portion is again removed from the plasmid and sequenced in conventional manner. To determine the minimal replicon deletions are made from each end of the desired portion by use of suitable anzymes if necessary by introduction of suitable restriction sites again in conventional manner.

It will appreciated that deletions, substitutions and additions (e.g. insertions) may be made in this sequence which will not affect its ability to act as an origin of replication and the invention is intended to cover sequences containing such deletions, substitutions and additions retaining this ability.

A plasmid is then constructed containing the minimal replicon, the marker gene and a multiple cloning site necessary for it to function as a vector for the introduction of genes of choice into B.t. strains. The thus constructed plasmid vector is then used as carrier for a desired gene which is subcloned into it and used to transform a B.t. strain. Transformants are screened using PCR with primers specific for individual crystal protein genes or other desired genes and bioassayed. Transformation with vectors according to the invention produces stable transformants which retain all their native crystal protein gene-containing plasmids.

While the main use of such vectors according to the invention will be to introduce crystal protein genes into B.t. strains which do not normally express them for broadening of spectrum and the like, it is not intended that their use be so restricted as they may be empolyed equally well for the introduction of other genes encoding proteins of interest.

The transformed B.t. strains may be used to combat insects by applying them in an insecticidally effective amount to the insects or their habitat. Suitable formulations and their preparation and preparation of the B.t. strains is carried out for instance as described in USP 4,797,279 (the contents of which in this respect are incorporated herein by reference).

The following examples illustrate the invention.

All experimental procedures referred to below as traditional or conventional are performed as outlined in Sambrook et.al. Molecular Cloning: A Laboratory Manual Second Edition. 1989. New York: Cold Spring Harbor Lab Press.

All restriction enzymes are supplied by Pharmacia LKB Biotechnology, Alameda, CA and New England Biolabs, Inc., Beverly, MA; digestions are performed according to manufacturer protocol. Taq DNA Polymerase

4

(amplitaq) is obtained from Perkin-Elmer Cetus, Norwalk, CT and PCR reactions are performed according to their recommendations.

The Supplier of all remaining enzymes used is Pharmacia LKB. Chemicals are supplied by Sigma Chemical Co., St. Louis, MO.

## EXAMPLE 1: Identification of minimal replicon from HD73 50MDa plasmid

### a) Isolation of plasmid DNA

An HD 73 strain (cf Lereclus et al. Mol. Gen. Genet. 191:307-313 obtainable from Institute Pasteur, Paris, France or NRRL, Peoria, IL 51604 NRRL B-4488) is grown in suitable culture medium and the plasmid DNA is purified by CsCl density gradient ultracentrifugation. Agarose gel electrophoresis of the plasmid DNA shows i.a. the presence of 50, 7.8, 5.6 and 5.2 MDa plasmids.

The 50 MDa plasmid is isolated from the other plasmids by sucrose gradient ultracentrifugation. Once isolated it is digested with Bgl II and the restriction fragments separated by preparative agarose gel electro-phoresis and isolated from the gel by electroelution.

### b) Preparation of erythromycin resistance gene:

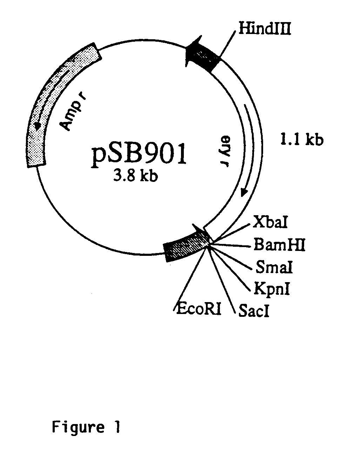

The Bacillus subtilis plasmid pIM13 (Mahler, et al. 198. J. Gen Microbiol 120: 259-263) is used as the source of an erythromycin resistance gene, ermC (Monod, et.al. 1986. J Bacteriol. 167: 138-147). The ermC gene fragment is cut out of pIM13 by a HindIII/ClaI double digest and separated from the rest of the plasmid by agarose gel electrophoresis. Once isolated the HindIII/ClaI ermC fragment is subcloned into HindIII/AccI-digested pUC18 (Pharmacia LKB Biotechnology, Alameda, CA) and selected for in E. coli DH5-alpha (Gibco BRL, Grand Island, NY). This construct is designated pSB901 (cf Fig. 1). From pSB901 the ermC gene is released by a HindIII/EcoRI or HindIII/BamHI double digest and as such carries with it sites from the MCS of pUC18. E. coli DH5-alpha cells containing pSB901 express erythromycin resistance but must be plated on LB plates containing 100 ug/ml erythromycin to prevent background growth of nontransformants which occurs on LB plates with 50 ug/ml.

### c) Preparation of 50 MDa plasmid subclones:

E. coli DH5-alpha serves as the host for subcloning the 50 MDa plasmid BglII fragments so that large amounts of plasmid DNA can easily be generated for eventual transformation of B.t. in order to isolate the BglII fragment containing the 50MDa plasmid origin of replication.

The BglII digest described above produces restriction fragments of approximately 35, 13.8, 10.2, 9.6, 4.2, and 3.3 kb as expected. The 13.8 kb BglII fragment is known to contain the cry IA(c) gene. Electroelution from gel slices yields good recovery of all fragments of 13.8 kb and below in size. All recovered BglII fragments are ligated separately to BamHI-digested pUC18 and E. coli DH5-alpha is transformed by conventional methods with the ligation mixes. Restriction digestion patterns of plasmid DNA are compared to the pattern expected based on Kronstad and Whiteley's work (J. Bacteriol. 160:95-102). Subclones containing the 3.3, 4.2, 9.6, and 13.8 kb BglII fragments are identified on the basis of their restriction pattern. The 13.8 kb subclone is verified by PCR analysis with crylA(c)-specific primers.

| Plasmid | Description |
|---------|-------------|
| pSB902 | 3.3 kb BglII fragment in pUC18 |
| pSB903 | 4.2 kb BglII fragment in pUC18 |
| pSB904 | 9.6 kb BglII fragment in pUC18 |
| pSB905 | 13.8 kb BglII fragment in pUC18 |

The ermC gene is inserted into these various plasmids to provide a selective marker for B.t. transformation:

The ermC gene is subcloned from pSB901 into pSB902 through pSB905. For example, a BamHI/HindIII ermC gene fragment from pSB901 is filled-in and then ligated into pSB904 at the pUC18 MCS PstI site which is blunted by T4 DNA Polymerase and treated with CIAP. E. coli DH5-alpha is transformed by traditional means with these ligation mixes. Restriction analysis confirms the presence of the ermC gene. The following plasmid designations are made:

| Plasmid | Description |
|---|---|
| pSB902.1 | 3.3 kb Bgl II fragment + ermC in pUC18 |
| pSB903.1 | 4.2 kb Bgl II fragment + ermC in pUC18 |
| pSB904.1 | 9.6 kb Bgl II fragment + ermC in pUC18 |
| pSB905.1 | 13.8 kb Bgl II fragment + ermC in pUC18 |

The 10.2 kb BglII fragment is subcloned directly into pSB901 which is digested with BamHI. E. coli DH5-alpha is transformed by traditional means with the ligation mix. Transformant plasmid DNA is analyzed by restriction digests. Restriction patterns confirm the presence of the 10.2 kb BglII fragment in the plasmids.

| Plasmid | Description |
|---|---|
| pSB906 | 10.2 kb BglII fragment in pSB901 |

d) **Identification of the Bgl II fragment containing the 50 MDa plasmid origin of replication:**

B.t. HD1 cry B (Stahly, et. al. Biochem Biophys Res Comm 1984. 3: 581-588); hereinafter cry B is separately transformed with pSB902.1, 903.1, 904.1, 905.1 and 906 by electroporation.

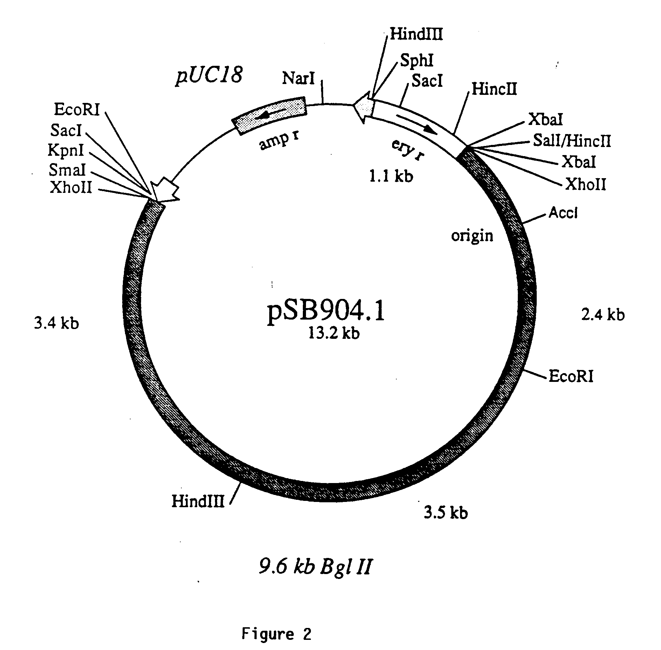

Only plasmid pSB904.1 (cf Fig. 2) yields erythromycin resistant transformants indicating that the 9.6 kb BglII fragment contains the origin of replication of the 50 MDa plasmid allowing it to be propagated in cry B. Restriction analysis verifies that the construct of correct size contains the 9.6 kb BglII fragment of the 50 MDa plasmid.

e) **Restriction Enzyme Analysis of pSB904.1:**

Restriction analysis with various enzymes is performed yielding the following results:

|  | pSB904.1 |
|---|---|
| AccI | 4* |
| BamHI | none |
| EcoRI | 2* |
| HincII | 4* |
| HindIII | 2* |
| KpnI | 1* |
| NarI | 1 |
| NcoI | none |
| PstI | none |
| SacI | 2* |
| SalI | 1* |
| SmaI | 1* |
| SphI | 1* |
| XbaI | 5* |
| XhoII | many |
| XmaI | 1* |

\* = one site is within the MCS which came from pUC18

**f) Removal of pUC18 sequences from pSB904.1:**

To remove pUC18 sequences, pSB904.1 DNA is digested with SphI and SmaI which cut at either end of the pUC18 vector and leave only MCS sequences behind. The restriction fragment containing only the 9.6 kb BglII fragment plus the ermC gene is purified away from the pUC18 vector by preparative agarose gel electrophoresis followed by electroelution. The SphI end of the fragment is treated with T4 DNA polymerase to produce a blunt end. The two blunt ends are ligated together with T4 DNA Ligase and cry B is transformed by electroporation with the ligation mix.

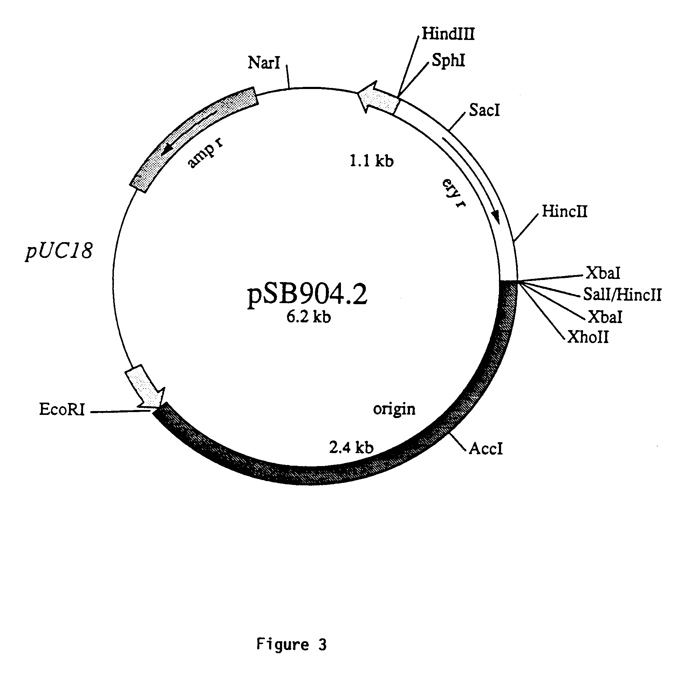

Transformants are screened for their plasmid content by agarose gel electrophoresis. This construct containing the 9.6 kb BglII fragment of the 50 MDa plasmid along with the ermC gene is designated pSB909 (cf Fig. 3).

| Plasmid | Description |
|---------|-------------|
| pSB909 | Deletes the pUC18 sequences from pSB904.1 (9.6kb BglII fragment plus the ermC gene remain) |

**g) Determination of the Location of the Origin of Replication on pSB909:**

To localize the area of the plasmid pSB909 which contains the origin of replication several constructs deleting various portions of the plasmid are made.

1. A HindIII digest is done on pSB904.1 resulting in 2 fragments.

The fragment containing about 6 kb of the B.t. portion plus the ermC gene is separated from the fragment containing pUC18 plus 3.4 kb of B.t. DNA by preparative agarose gel electrophoresis. The ermC containing fragment is removed from the gel slice by eletroelution and self-ligated to recircularise.

2. A HindIII/EcoRI double digest is done on pSB909 resulting in 2 fragments. The large fragment containing the ermC gene is separated from the small 3.5 kb B.t. DNA fragment by preparative agarose gel electrophoresis. After electroelution, the large fragment is filled-in using Klenow to produce blunt ends. These blunt ends are self-ligated to reclose the vector.

3. An EcoRI/SalI double digest is done on pSB909 resulting in 2 fragments. The large fragment containing the ermC gene is separated from the small 2.4 kb B.t. DNA fragment by preparative agarose gel electrophoresis. After electroelution, the large fragment is filled-in using Klenow to produce blunt ends which are self-ligated to recircularise. In this way, each portion of pSB909 is separately deleted.

To determine which construct retains the origin of replication, the various ligation mixes are used to transform cry B. Transformants are not obtained when the EcoRI/SalI 2.4 kb fragment is deleted thereby localising the origin to that piece of DNA. This is verified by obtaining transformants with the other 2 deletion constructs.

| Plasmid | Description |
|---------|-------------|
| pSB909.1 | removes the EcoRI/HindIII 3.5 kb piece from pSB909 |
| pSB909.2 | lacks the HindIII portion of 3.4 kb in pSB909 |

**h) Construction of 909.3**

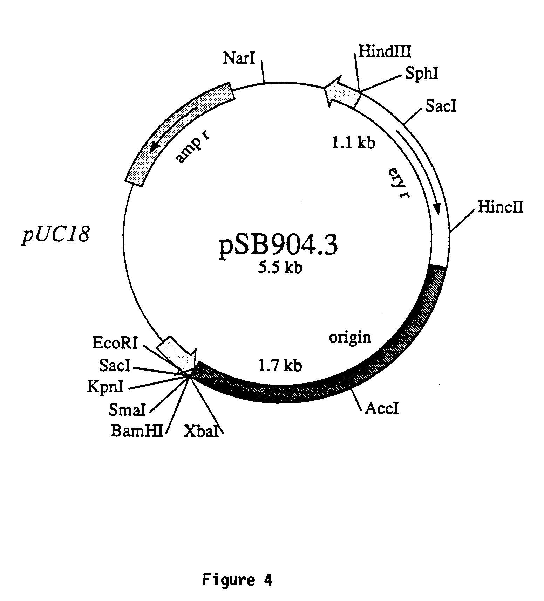

In order to verify that the 2.4 kb portion of pSB909 contains the origin of replication as suggested by the deletion studies, a new plasmid is constructed that contains only the 2.4 kb portion of pSB909 along with the ermC gene. Plasmid DNA from pSB904.1 is digested with both EcoRI and HindIII to produce restriction fragments of approximately 3.6, 3.5, 3.4, and 2.7 kb (the pUC18 portion). This mix of restriction fragments is filled-in using Klenow to produce blunt ends and then ligated together to recircularise. The ligation mix is used to transform cry B by electroporation. Transformants obtained are screened for their plasmid content by agarose gel electrophoresis. The 3.6 kb fragment, which is made up of the 2.4 kb B.t. portion along with the ermC gene, is capable of replication confirming that it contains the origin of replication of the 50 MDa plasmid. This construct is designated as pSB909.3 (cf Fig. 4).

| Plasmid | Description |
|---|---|
| pSB909.3 | Contains the 2.4 kb portion from the 9.6 kb 50 MDa Bgl II fragment along with the ermC gene |

## i) Sequencing of 909.3

In order to sequence the 2.4 kb portion containing the origin of replication from the 50 MDa plasmid, this portion is removed from pSB904.1 by digestion with EcoRI and SalI. The EcoRI/SalI fragment is subcloned into the E.coli plasmid vector pSport (Gibco BRL, Grand Island, NY).

Sequencing is performed according to the dideoxy method according to manufacturer's recommendations (T7 DNA Polymerase Sequencing Kit, Pharmacia LKB Biotechnology, Alameda, CA). Sequencing results indicate that the replication fragment is 2392 bp in size.

## j) Determination of minimal replicon

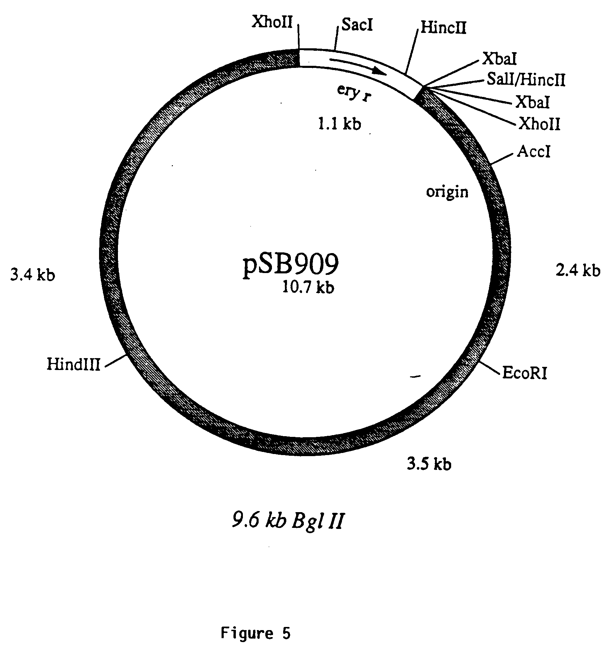

In order to delete various portions from the EcoRI end, designated as nucleotide number 2392, of the B.t. replicon fragment subcloned into pSport, Exo III is used according to the manufacturers recommendations. ("Erase-A-Base" kit, Promega, Madison, WI). By sequence analysis the ending nucleotide of each deletion is determined. The results obtained are given below in Table 1 and are illustrated in Fig. 5. Plasmid DNA of each ExoIII deletion subclone is digested with both HindIII and XbaI. A HindIII/XbaI fragment carrying the ermC gene is ligated to each ExoIII subclone. The E.coli strain DH5-alpha is transformed according to traditional methods with the separate ligation mixes. Plasmid DNA from transformants is analyzed by restriction digestion with HindIII and XbaI to confirm the presence of the ermC gene within a particular ExoIII deletion subclone. Transformation of cry B by electroporation is done with plasmid DNA from each ExoIII subclone to determine which is still capable of replication.

### Table 1

| Deletion end point | cry B replication |
|---|---|
| 2281 | Yes |
| 1912 | Yes |
| 1626 | No |
| 1500 | No |
| 1383 | No |
| 1190 | No |

Plasmids capable of replication in cry B are analyzed by agarose gel electrophoresis. Thus, 479 bases can be deleted from the EcoRI end of the replicon fragment without destroying the capacity for replication. Accordingly, one end of the minimal required DNA for replication lies between base pairs 1626 and 1912.

To determine how much DNA can be removed from the BglII end of the replicon fragment, designated as nucleotide number 1, without losing replication functions, deletions are introduced by substitution of portions of the fragment. The plasmid pSB904.1 is digested with EcoRI to produce two fragments, one containing pUC18 + ermC + the 2.4 kb replicon fragment.

The mixture is ligated to circularise the fragments and E. coli DH5-alpha is transformed with the ligation reaction according to standard protocol. Transformant plasmid content is analysed by agarose gel electrophoresis following digestion with EcoRI. This construct, which contains the ermC gene and the 2.4 kb origin of replication fragment in pUC18, is designated pSB904.2 (cf Fig. 2A). Since no convenient restriction site exists within the first 200 bases of the replicon fragment, a PCR primer, designated BT113 (SEQ ID NO:1 in the accompanying SEQUENCE LISTING), is designed to introduce an AvrII site at nucleotide position 123 by a single base change which is underlined below. A second primer, designated BT898.rev (SEQ ID NO: 2 in the accompanying SEQUENCE LISTING), is designed to allow amplification of a portion of the 2.4 kb replicon fragment from nucleotide number 113 through 898. The primer sequences are shown below.

| Primer | Sequence | Position |
|---|---|---|
| BT113 | 5' GAA TCA AGC CTA GGC ACT AGG TTG 3' | 113-137 |
| BT898.rev | 5' CTC AAT TGC TAG ATG CCA TTT GTG 3' | 898-875 |

This primer pair is used in PCR with pSB904.2 plasmid DNA serving as template to produce a 786 bp fragment. The PCR fragment is digested with AvrII and AccI generating a fragment corresponding to nucleotide number 123 through 823 which is gel purified. The plasmid pSB904.2 is digested with XbaI and AccI and following gel purification is ligated to the AvrII/AccI PCR fragment. AvrII and XbaI have compatible cohesive ends to allow the insertion of the PCR fragment into the pSB904.2 XbaI/AccI vector. This insert creates a new 5' end of the replicon fragment which lacks 122 bases of the original fragment. Alternatively, the PCR fragment is digested with DraI and AccI generating a fragment corresponding to nucleotide number 291 through 823. Following phenol/chloroform extraction and ethanol precipitation, this DraI/AccI PCR fragment is ligated directly to pSB904.2 which has been digested with XbaI, treated with Klenow to blunt the ends, followed by digestion with AccI, phenol/chloroform extraction and ethanol precipitation. The DraI blunt end will ligate to the blunted XbaI end to allow insertion of the DraI/AccI PCR fragment into the pSB904.2 vector resulting in a deletion of 290 bp from the original "BglII" end.

Both ligation mixes are separtely used to transform E. coli DH5-alpha according to traditional methods. Transformants are screened by PCR analysis using primers which surround the deletion points and confirmed by restriction digestion analysis. Plasmid DNA from each deletion construct is used to transform cry B by electroporation to determine whether the deletion constructs are still capable of replication.

| Deleted portion | cry B replication |
|---|---|
| 1-122 | Yes |
| 1-291 | No |

The deletion construct still capable of replication in cry B is analysed by agarose gel electrophoresis and confirmed by PCR analysis. This plasmid is designated pSB904.2δA3. Thus, this end of the minimal replicon lies between basepairs 122-291.

To test whether additional nucleotides can be deleted from the EcoRI end of the replicon, pSB904.2δA3 is digested with HindIII and AflIII to remove the pUC18 portion and retain basepairs 123-1843 of the B.t. replicon as well as the ermC gene. This HindIII/AflIII fragment is subcloned into pUC18 in DH5-alpha and following confirmation is used to transform cry B to determine if the deletion is capable of replication. Transformants obtained are analysed for their plasmid content by agarose gel eletro-phoresis. This plasmid is designated pSB904.3 (cf Fig. 2B).

| Deletion Construct | cry B replication |
|---|---|
| 123-1843 | Yes |

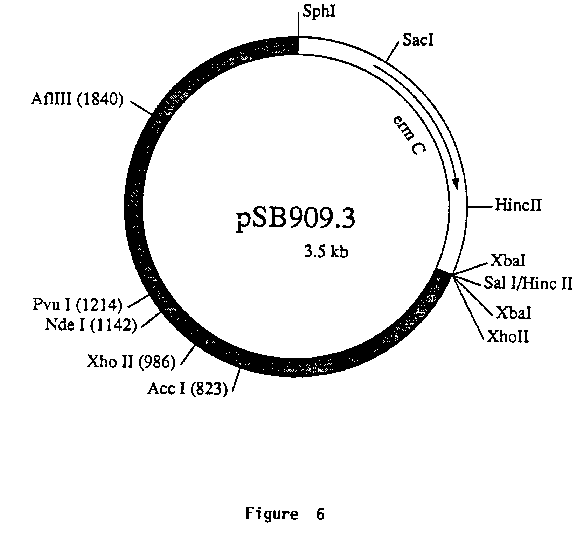

To ensure that pUC18 sequences are not necessary for the replication function of deletion constructs, a construct lacking the pUC18 portion is obtained by filling in the ends of the HindIII/AflIII fragment described above from pSB904.2δA3 with Klenow and ligating to circularise. The ligation mix is used to transform cry B by electroporation. Transformants are analyzed for their plasmid content by agarose gel electrophoresis and confirmed by PCR and restriction digestion analysis. This plasmid, which is composed of the B.t. replicon nucleotides 123-1843 and the ermC gene, is designated pSB909.4 (cf Fig. 6) and delineates the minimal replicon. The sequence of the minimal B.t. replicon is given as SEQ ID NO:6 in the accompanying SEQUENCE LISTING.

EXAMPLE 2:

## Transformation of B.t. with plasmid vector containing minimal replicon

### a) Multiple-Cloning Site Insertion

In order for this minimal replicon to be useful as a cloning vector, a multiple-cloning site is introduced into it. The MCS is constructed of two oligonucleotides, whose sequence is indicated below, which when hybridised form an AflIII and a HindIII sticky end.

| Oligo | Sequence |
|---|---|
| MCS1 | 5′ CATGTGAATTCCGCGGTACCCGGGGATCCTCTAGAGT CGACCTGCAGA3′ |
| MCS2 | 5′ AGCTTCTGCAGGTCGACTCTAGAGGATCCCCGGGTACC GCGGAATTCA3′ |

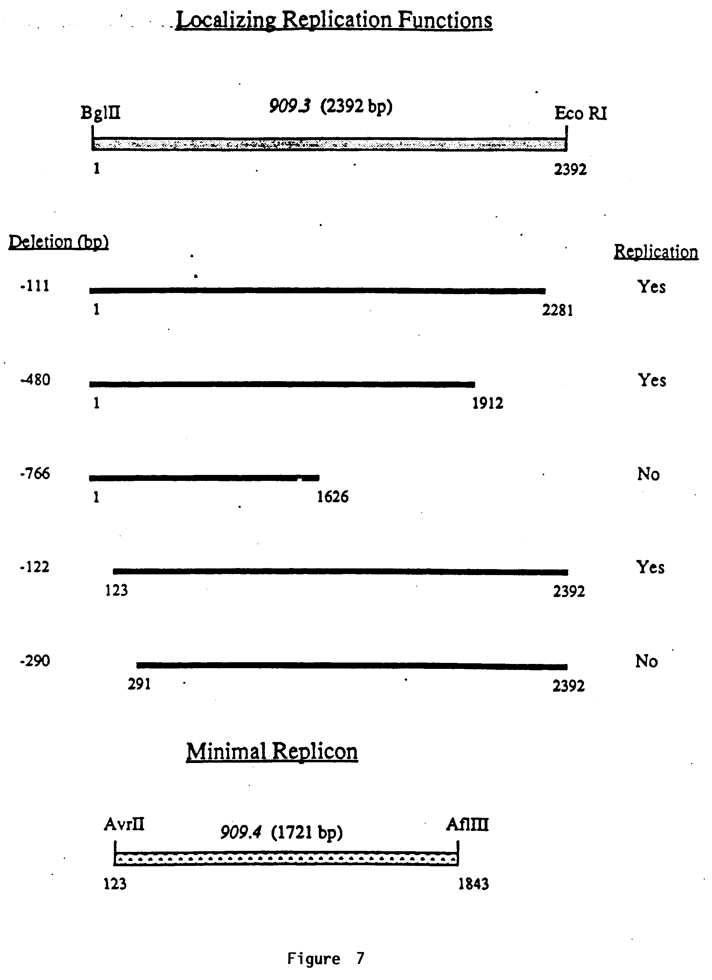

Oligonucleotide MCS1 and MCS2 are given as SEQ ID NO 3 and SEQ 10 NO 4 respectively in the accompanying SEQUENCE LISTING. These oligonucleotides are kinased separately with T4 Polynucleotide Kinase, mixed and heated to boiling, allowed to cool gradually to room temperature, and ligated in 1000-fold molar excess with the HindIII/AflIII fragment from pSB904.2δA3 using T4 DNA Ligase. The ligation mix is used to transform cry B by electroporation. Transformants are screened by restriction digestion to verify the presence of the MCS within the plasmid. This construct is designated pSB909.5 (cf Fig. 7).

### b) Cry IIIA gene isolation

The cryIIIA delta-endotoxin gene is isolated and cloned from B.t. tenebrionis. The gene is located on a 90 MDa plasmid. Total DNA from B.t. tenebrionis is isolated by cell lysis in SDS and precipitation of contaminants with NaCl, followed by dialysis and precipitation of DNA in ethanol. Total DNA preparations contain the 90 MDa plasmid carrying the cryIIIA gene. A 42 base probe, Tenebr 1 (SEQ ID NO:5 in the accompanying SEQUENCE LISTING), was synthesized for use in Southern blot detection of the cryIIIA gene. The probe is homologous to a sequence near the ATG start codon. Sequence of the probe is derived from the published cryIIIA sequence (Sekar, et al. 1987. Proc. Natl. Acad. Sci. 84: 7036-7040.)

| Oligo | Sequence |
|---|---|
| Tenebr1 | 5′ACT ACT GAA AAT GAG GTG CCA ACT AAC CAT GTT CAA TAT3′ |

Total DNA is digested with HindIII, separated by gel electrophoresis in 0.6% agarose in 1xTBE buffer. Southern blotting of the digested DNA transferred to nylon filters with the probe Tenebr1, upon autoradiography, exhibits a hybridising HindIII fragment of 3.0 kb. Total DNA is digested to completion with HindIII. The resulting fragments are separated by gel electrophoresis. A portion of the DNA, corresponding to 2.5-3.5 kb in size, is isolated on DEAE membrane, eluted off and resuspended in 100ul TE. A partial library is then created by ligating the isolated HindIII fragments into HindIII cut pTZ18R (Pharmacia LKB Biotechnology), treated with bacterial alkaline phosphatase. Library grade BRL competent DH5-alpha cells are transformed using 1 μl ligation reaction to 25 μl competent cells and recombinants are selected on LB plates containing Ampicillin at 75 μg/ml, X-gal at 100 μg/ml, and IPTG at 1mM. The resulting colonies are screened by colony DNA hybridisation to the probe, Tenebr1. Positive colonies are grown up and plasmids are isolated and examined by restriction analysis. The plasmid clone producing the correct restriction fragments and containing an insert of the appropriate size is named pSB100. Further evidence that pSB100 contains the cryIIIA gene is obtained from Southern blot analysis with the Tenebr1 probe.

### c) Subcloning of CryIIIA gene into pSB909.5

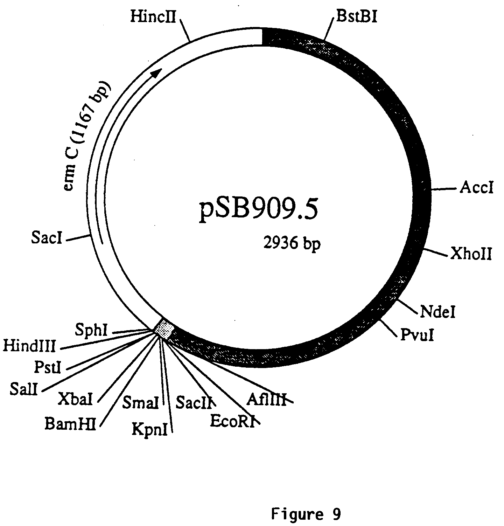

Plasmid DNA is isolated in large scale from the cry B strain containing pSB909.5 by alkaline lysis proceeded by incubation of the cells at 37°C for 30-60 minutes in a solution of 25mM Tris pH 8.0, 25% Sucrose, 25mM EDTA and followed by Potassium Acetate precipitation of chromosomal DNA. The supernatant is loaded directly onto a Qiagen tip-100 column (Qiagen Inc., Chatsworth, CA) to purify the plasmid DNA. The plasmid vector DNA is then digested to completion with HindIII followed by treatment with CIAP. The HindIII and CIAP are removed by extraction with Strataclean Resin (Stratagene, La Jolla, CA). The cryIIIA gene is isolated from pSB100 by digestion with HindIII which releases the gene intact from the pTZ18R vector. The HindIII fragment containing the cryIIIA gene is isolated by preparative agarose gel electrophoresis followed by extraction from the agarose with Qiaex (Qiagen Inc., Chatsworth, CA). The isolated cryIIIA HindIII fragment is ligated to the HindIII digested pSB909.5 using T4 DNA Ligase and a vector to insert ratio of 1:5. The ligation mix is used to transform cry B by electroporation. Transformants are selected on LB plates containing 50 μg/ml at 30°C. Colonies are screened for their plasmid content. Plasmids of the correct size are analysed by PCR with primers specific for the cryIIIA gene. The construct made up of the cryIIIA HindIII gene fragment subcloned into the HindIII site of pSB909.5 is designated pSB922 (cf Fig. 9).

### d) Transformation of B.t. kurstaki

Plasmid DNA is isolated in large scale from the cry B strain carrying pSB922 as indicated above. The purified plasmid DNA is used to transform by electroporation a B.t. kurstaki strain which contains the CryIA(a), CryIA(b), CryIA(c), and CryIIA genes. Transformants are selected as above. Isolated colonies are analysed for their plasmid content. Transformants are screened for their cry gene content by PCR with primers that are specific for each crystal gene. The introduction of the cryIIIA-containing pSB909.5 into this B.t. kurstaki strain does not result in the displacement of any of the native crystal protein gene-containing plasmids.

Bioassays are performed with transformed cultures allowed to sporulate in suitable media. Activity is expected against both lepidopteran and coleopteran species. Insects used in bioassay include Leptinotarsa texana, Trichoplusia ni, Heliothis zea, and Spodoptera exigua.

After over ten generations of vegetative growth in the absence of antibiotic selection, the plasmid vector carrying a crystal gene is stably maintained within the transformed cell. Thus, this B.t. plasmid vector is useful for subcloning various genes and introducing them stably into various B.t. strains.

Combinations of the generic, subgeneric and species claims which follow and of the various preferences referred to in this specification are especially preferred.

SEQUENCE LISTING

(1) GENERAL INFORMATION:

   (i) APPLICANT: Sandoz Technology Ltd.

  (ii) TITLE OF INVENTION: NOVEL VECTORS

 (iii) NUMBER OF SEQUENCES: 6

  (iv) CORRESPONDENCE ADDRESS:
     (A) ADDRESSEE: Sandoz Technology Ltd.
     (B) STREET:
     (C) CITY: Basel
     (D) STATE: Basel-Stadt
     (E) COUNTRY: Switzerland
     (F) ZIP: CH-4002

   (v) COMPUTER READABLE FORM:
     (A) MEDIUM TYPE:
     (B) COMPUTER:
     (C) OPERATING
     (D) SOFTWARE:

  (vi) CURRENT APPLICATION DATA:
     (A) APPLICATION NUMBER:
     (B) FILING DATE:
     (C) CLASSIFICATION:

(viii) ATTORNEY/AGENT INFORMATION:
     (A) NAME: Crawley, Patrick E..
     (B) REGISTRATION NUMBER: 51040
     (C) REFERENCE/DOCKET NUMBER: 133-0693

  (ix) TELECOMMUNICATION INFORMATION:
     (A) TELEPHONE: 061 324 4796
     (B) TELEFAX:   061 322 7532

(2) INFORMATION FOR SEQ ID NO:1:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO:1:

GAATCAAGCC TAGGCACTAG GTTG      24

(2) INFORMATION FOR SEQ ID NO:2:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO:2:

CTCAATTGCT AGATGCCATT TGTG      24

(2) INFORMATION FOR SEQ ID NO:3:

     (i) SEQUENCE CHARACTERISTICS:
         (A) LENGTH: 48 base pairs
         (B) TYPE: nucleic acid
         (C) STRANDEDNESS: single
         (D) TOPOLOGY: linear

     (ii) MOLECULE TYPE: DNA (genomic)

     (iii) HYPOTHETICAL: NO

     (iv) ANTI-SENSE: NO

     (xi) SEQUENCE DESCRIPTION: SEQ ID NO:3:

CATGTGAATT CCGCGGTACC CGGGGATCCT CTAGAGTCGA CCTGCAGA     48

(2) INFORMATION FOR SEQ ID NO:4:

     (i) SEQUENCE CHARACTERISTICS:
         (A) LENGTH: 48 base pairs
         (B) TYPE: nucleic acid
         (C) STRANDEDNESS: single
         (D) TOPOLOGY: linear

     (ii) MOLECULE TYPE: DNA (genomic)

     (iii) HYPOTHETICAL: NO

     (iv) ANTI-SENSE: NO

     (xi) SEQUENCE DESCRIPTION: SEQ ID NO:4:

AGCTTCTGCA GGTCGACTCT AGAGGATCCC CGGGTACCGC GGAATTCA     48

(2) INFORMATION FOR SEQ ID NO:5:

    (i) SEQUENCE CHARACTERISTICS:
        (A) LENGTH: 39 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO


    (xi) SEQUENCE DESCRIPTION: SEQ ID NO:5:

ACTACTGAAA ATGAGGTGCC AACTAACCAT GTTCAATAT        39


(2) INFORMATION FOR SEQ ID NO:6:

    (i) SEQUENCE CHARACTERISTICS:
        (A) LENGTH: 1721 base pairs
        (B) TYPE: nucleic acid
        (C) STRANDEDNESS: double
        (D) TOPOLOGY: linear

    (ii) MOLECULE TYPE: DNA (genomic)

    (iii) HYPOTHETICAL: NO

    (iv) ANTI-SENSE: NO


    (xi) SEQUENCE DESCRIPTION: SEQ ID NO:6:

CTATGCACTA GGTTGATATT TCATAAAAAA AAAGATAGAA TATATAACAT AATATATATA  60

ATTAAAAAAG AGATTTATGA TATAGTTATT TCATATAAAC AGGAAATTAA GTTCAATTCA  120

TTTAAAAAAG ACAAAAAATA AAACTCCACC CACCGTTTTC AATTGATTCG AAGTTTGGTG  180

ACTAGATCAA TTGAAAACCC ACGTTAGCAG AGTTTTTTTA CAAAATTAAT ATTATGGTTA  240

AATTATATCA TAACTAATGA AGTTTTGTGA ATAAAAAACT CGCTAATTTT CCGATATCGG  300

GCTGGAAATA TTAGGGAGGT TTTTTCGTGT TATTAAAAAA TTTTATATTA AGCAAAGCAA  360

```
AACGAGAACT AAGTCCTCAA CTGGATCGTA TATCTACTAA ACCAGGAAAT ATTAATAAAG  420

CCGTAAAAAA AATTCTAAAG GAGATTGATA TCATTCTTTT AGAAGACCAG GGCTTTACTA  480

CTGAAGACAT TTCTTTACAA ACTCCTCGTA AGTACAAAGG ACTTGAAACA ATTTTATCTT  540

ATCTTGTTCA CGAAGGTTTT TCTTGTGTGA AACAGGATGT GATAGCCCAA AAAGCGGGAA  600

TTTCAAAACC CCTTATCAAT GAAACTCTTT CTTGGCTAGA AAAACTTGGT ATCTGCCACC  660

AAATTAGAAA TCGTAAAGCT GGAAAGAAAG GTCCTTCTGT ATACATTTTA ACAATACATA  720

ATAATTATCA AAAAATCATA GATTACTTTA AACACAAATG GCATCTAGCA ATTGAGATAA  780

CAGAAACTAT CTCAAACCTA ATATCCAAGT GGACTCTATT AAAAGAAAAA AAAGAAGAAG  840

TTACAGACGA ACAAGTAGAT ACAGATCCAA AACAAGAAGA AGCTAATGAA CAAAGTGAAG  900

AACCTAAAGA CTATTTTGCT AGAAAAGAAG ATTTCAATGA ATATTTAAGT AAAGATCAAC  960

AAAGAGCATG GCACTATATT ATGAGTTCTC CATTTACTAA TCTGTCTGAA AAAGATGCAT 1020

ATGCAATTGC AAATAGAATG CCACCAGATA TCGACAGAGA TGCTTGGTAT TTCTTCAGAC 1080

AAGCTGCCGA TCGTTTTGAA GCTAGTAAAG CTGATAAGAG TAATGCTGCT TATTTCATTG 1140

AAATATTTAG CCAAAACTAT AAATCTTATT TAAAACGTAA AAAAGAAGAA GCTGAAAAAT 1200

ATGTTTCATC TTTATCAAAA CCAAAACAAA AGTTAATAAT TTACGATTTT ATCAAAGGGG 1260

AATAATCTTG CTTAAAATCG CCATTTTTAT AGCCTTGTTT AAAAATTATA GCAAAACAAG 1320

GCTATTTGTC ATGCGCTTAT ATTATTTCCT AAATACCTCT CATACTTCAC ATCCAAATCA 1380

ATTAAAAATA ACAGTTAATT TTTAGCATAT AATGGAAAAA CATAAAAAGA TAACCTACTA 1440

AAATTGCCGC AATAAATGAA CCGAAAATTC AGATACCTCC TTTGTTTCTA CCAAATAACT 1500

ACTAAGAAAA AAAATCAAAC TTCTTGTTAG AGTAAGGGTT AAGTGCCATT TTTAATTTCT 1560

AATGTATTTA ATGGTTTTAA TTTTTAAAAC AAGATTTATT AAATAAAAGA ATCTTATCAT 1620

AATTCATCAA ATTTTTTTAA ACACCTTTTG TCATTCTCCC TACTGTTTCC CTAATCCTCG 1680

CTAAAATAGA CACCCAATAT GTAAATGTCA AGATAAACAT G                      1721
```

## Claims

1.  A DNA vector for use in transformation of Bacillus thuringiensis cells comprising an origin of replication from Bacillus thuringiensis kurstaki HD73 substantially free of DNA with which it is normally associated and which is not necessary for replication.

2.  A DNA vector according to Claim 1 wherein the DNA contained therein is predominantly Bacillus thuringiensis DNA.

3.  A DNA vector according to Claim 1 or 2 wherein the origin of replication has the nucleotide sequence of SEQ ID NO:6 and homologues thereof which involve deletions, additions or substitutions which do not affect the ability to function as an origin of replication.

4. A DNA vector according to Claim 1, 2 or 3 which additionally comprises a means for selecting hosts transformed by the vector and a cloning site.

5. A DNA vector according to Claim 4 which additionally contains integrated in the cloning site a region of DNA which enables gene expression in Bacillus thuringiensis with one or more DNA sequences which upon expression encode one or more desired Bacillus thuringiensis crystal proteins or other desired proteins.

6. A method of producing in a Bacillus thuringiensis strain a desired exogenous protein which comprises transforming cells of said Bacillus thuringiensis strain with a vector according to Claim 1 or 2 which comprises a gene coding for expression of the desired exogenous protein and propagating said transformed cells in a suitable manner to effect expression of the gene encoding the desired protein.

7. A method as claimed in Claim 6 wherein the DNA sequence encodes a Bacillus thuringiensis crystal protein.

8. A Bacillus thuringiensis strain which has been transformed with a DNA vector according to claim 1, 2 or 5.

9. An insecticidal composition comprising a Bacillus thuringiensis strain as claimed in Claim 8.

10. An insecticidal composition comprising a Bacillus thuringiensis strain as claimed in Claim 9.

11. An origin of replication isolated from Bacillus thuringiensis kurstaki HD73.

12. An origin of replication according to Claim 11 which has the nucleotide sequence shown in SEQ ID NO:6 and homologues thereof which involve deletions, additions or substitutions which do not affect the ability to function as an origin of replication.

Figure 1

Figure 2

Figure 3

Figure 4

*9.6 kb Bgl II*

Figure 5

Figure 6

EP 0 537 105 A1

## Localizing Replication Functions

Figure 7

24

Figure 8

Figure 9

Figure 10

**European Patent Office**

## EUROPEAN SEARCH REPORT

Application Number

EP 92 81 0549

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | APPLIED AND ENVIRONMENTAL MICROBIOLOGY vol. 56, no. 11, 1990, WASHINGTON D C pages 3420 - 3428 BAUM J A; COYLE D M; GILBERT M P; JANY C S; GAWRON-BURKE C 'NOVEL CLONING VECTORS FOR BACILLUS-THURINGIENSIS' * the whole document * | 1,6,8,9, 11 | C12N15/75 C12P21/00 C12N15/32 A01N63/00 //(C12P21, C12R1/07) |
| D,Y | EP-A-0 433 945 (SANDOZ LTD. ET AL.) * the whole document * | 1,6,8,9, 11 | |
| A | | 2,4,5,7, 10 | |
| P,D, X | JOURNAL OF BACTERIOLOGY vol. 173, no. 17, 1991, BALTIMORE US pages 5280 - 5289 Baum, James A.; Gilbert, M. Pearce 'Characterization and comparative sequence analysis of replication origins from three large Bacillus thuringiensis plasmids' * the whole document * | 1-3,8 | |
| P,X | WO-A-9 118 102 (ECOGEN INC.) * the whole document * | 1-3,8 | TECHNICAL FIELDS SEARCHED (Int. Cl.5 ) C12N C07K A01N |

The present search report has been drawn up for all claims

| Place of search | Date of completion of the search | Examiner |
|---|---|---|
| BERLIN | 11 JANUARY 1993 | GURDJIAN D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)